# EUROPEAN PATENT SPECIFICATION

(11) **EP 1 307 145 B1**
(45) Date of publication and mention of the grant of the patent: **28.11.2007**
(21) Application number: 00953957.8
(22) Date of filing: 10.08.2000
(51) Int. Cl.: A61B 17/12

(54) **INTRAVASCULAR DELIVERY SYSTEM**
INTRALUMINALES ABGABESYSTEM
SYSTEME D'ADMINISTRATION INTRAVASCULAIRE

(43) Date of publication of application: 07.05.2003
(60) Divisional application: 07019614.2
(73) Proprietor: Micrus Corporation, Sunnyvale, California 94085 (US)
(72) Inventor: KURZ, Daniel, R., Monterey , CA 93940 (US)
(74) Representative: McLeish, Nicholas Alistair Maxwell
(86) International application number: PCT/US2000/021928
(87) International publication number: WO 2002/013705

(56) References cited:
- EP-A- 0 941 700
- WO-A-00/12015
- WO-A-97/01368
- WO-A-98/02100
- WO-A-99/32037
- US-A- 5 609 608
- US-A- 6 102 932

## Description

### BACKGROUND OF THE INVENTION

### Field of the Invention:

This invention relates generally to therapeutic placement of interventional medical devices into the vasculature of the human body. More particularly, this invention concerns a placement system using a catheter with a tip formed of yieldable material to grip and thereby releasably capture a portion of an endoluminal device to be dislodged at a desired location within the body by a device for dislodging the endoluminal device from the catheter tip.

### Description of Related Art:

A type of interventional medical device known as an endoluminal coil is used for a wide variety of therapeutic purposes including the treatment of intracranial vascular aneurysms. A vascular aneurysm is often formed as the result of an abnormal dilation of a blood vessel which weakens the arterial wall and allows it to expand into an adjacent body tissue or cavity. Intracranial aneurysms may be treated to prevent rupturing by placing endoluminal coils through the neck of an opening from the vessel into the interior cavity of the aneurysm. After placement, the coils pose a physical barrier, reducing blood flow into the aneurysm and promoting the formation of an embolus in the aneurysm cavity. The embolus formation in the aneurysm cavity further impedes blood flow into the aneurysm and reduces the blood pressure against the aneurysm wall, thus reducing the possibility of a rupture.

One known method for delivering coils into an intracranial aneurysm involves the use of a catheter and a guidewire with a detachable tip shaped in the form of a coil. Microcatheters are known, for example, that allow for navigation into cerebral arteries and entry into intracranial aneurysms. The catheter is guided through the vasculature using a guidewire until it reaches the desired location. The tip of the guidewire is then detached and the coils are pushed into the aneurysm until they occlude at least a portion of the interior of the aneurysm. Although generally effective, this technique has limitations on the accuracy for precision placement of embolic coils in intracranial aneurysms. It would be particularly desirable to have a simple delivery system which allows for precise positioning of embolic coils and virtually instantaneous release once the coils are in place.

One approach which provides for greater accuracy of placement involves attaching a coil to the end of the a guidewire and maneuvering the guidewire to place the embolic coil in the desired location and then releasing the coil from the guidewire. Since the physician has control of the guidewire and the coil is firmly attached to the distal end of the pusher, it is possible to achieve a much higher degree emplacement accuracy with this approach. However, to implement this approach, the delivery system must include a release mechanism which can be used to easily decouple the coil from the guidewire while inside tiny blood vessels. A variety of release mechanisms have been proposed for this purpose.

WO 99/32037 discloses a coil-delivery device for delivery of a vaso-occlusion aid to a vascular target site. The device includes a wire adapted to be slidably received within the lumen of a catheter or introducer, the wire having at its distal end a stiff wavy wire segment adapted to frictionally and releasably engage a vaso-occlusion coil by the end-region inner lumen to the vaso-occlusion coil. The coil is positioned and the device retracted, for example bringing the engaged end of the coil into contact with a valve at the distal end of the catheter and dislodging the coil from the wavy wire segment.

EP-A-0941700 discloses an alternative technique for deploying an embolic coil. The device of EP-A-0941700 comprises a positioning catheter having a distal tip for retaining the embolic coil which when pressurized with a fluid expands outwardly to release the coil.

In one known technique for deploying an endoluminal device, endoluminal coils may be released through electrolytic dissolution of a connecting segment between the coil and the distal tip of the guidewire. This method typically involves the application of a positive direct current for a predetermined amount of time which results in the dissolution of a stainless steel connector which holds the coil to the guidewire. Although this method has met with considerable success, the procedure has significant disadvantages. Because the use of electrolytic dissolution is slow and unreliable, the delivery of the devices can be very time consuming and therefore very costly. The increased surgery time also creates a higher risk for the patient. In addition, the secondary effects of dissolving a stainless steel wire in the blood could possibly be detrimental to the patient. For these reasons, a simpler, faster, safer and more reliable method of delivering the devices is needed.

Detachable coil assemblies are also known that use a threaded coupling such that the coil is released when the guidewire is rotated. Another conventional technique uses a heat-releasable adhesive bond to separate the coils from the distal end of the catheter. When laser energy is transferred via a fiber optic cable to the connector, the connector is heated, thereby releasing the heat-sensitive adhesive bond between the connector and coil.

One known implant delivery assembly is activated thermally, and includes a coupling portion made of a shape memory material that interlockingly engages the implant when the shape memory material is in one configuration, and releases the implant in another configuration. The implant is detachably coupled to a pusher formed of shape memory material that allows thermal activation of the decoupling mechanism. The coupling portion is constructed with a deformed shape for holding the implant to the pusher, and apre-set shape that provides release of the implant when the thermal activation is provided. The coupling portion of the pusher is heated by passing an electric current between the pusher and the body of the patient.

An example of a delivery system for delivering a stent where upon heating the stent is released is provided in WO 98/02100 where a bush may be made of a shape memory alloy. The bush engages at one end with the wire of the stent and at the other with a pusher wire. Upon heating the portion of the bush engaging the wire of the stent expands, thereby releasing the wire of the stent, at the other end the bush remains engaged with the pusher wire. Alternatively the bush may simply be engaged with the two wires by friction. When the wire of the stent enters the blood it expands to adopt its expanded structure and the bush is pulled off the wire of the stent.

Another example of the use of shape memory material in an implant delivery device is provided in WO 00/12015 where an introducer for retaining an implant placed within an implant delivery device is disclosed. The device includes a pusher for displacing the implant and a tube and a clamp. The interior of the tube defines a passage for guiding the movement of the pusher. The clamp is made of a shape memory material disposed in the passage about the pusher. The clamp is normally operative to grip the pusher and is further operative in response to an elevated temperature to release the pusher for movement relative to the tube.

Another detachable embolic coil assembly is known that uses interlocking clasps that are used in a surgical instrument for delivering an embolic coil to a selected site within the vasculature of the human body.

Yet another known embolic coil assembly includes a ball that is forced through an aperture in a socket on the distal end of a pusher to release the coil. After a catheter is inserted and navigated through the vessel, and the coil is in place, a plunger is advanced to press the ball and its coil into the target site.

Some conventional vasoocclusive devices are operated by pulling or jerking the catheter tip from an inflatable balloon, thus potentially compromising the position of the implant. One such device provides for an endovascular wire and tip that can be sep arated from the holding wire mechanically or electrolytically for the formation of thrombus in blood vessels. However, such devices that release the interventional device by mechanically breaking an intermediate section between the catheter tip and balloon can potentially leave broken or jagged ends that could injure the vasculature.

One conventional releasable balloon catheter used to embolize vascular lesions has a tubular portion made of a material such as a hydrophilic polymer located between the catheter and the balloon that can be broken by torsion of the tubular portion. The tubular portion can be melted by heating the tubular portion, or can be dissolved in the blood when heated, and electrodes are provided for heating the tubular portion. Another conventional technique for separating a balloon from a balloon catheter involves the melting and breaking of a connecting member made from polyvinyl alcohol or trans-polyisoprene between the balloon and the catheter body when power is supplied to electrodes provided for heating the connecting member. When the connecting member is heated to temperatures of about 70°C and slight tension is applied, the balloon can be separated from the main catheter body. However, such devices that release the interventional device by melting or dissolving the intermediate section between the catheter tip and balloon can also potentially release undesirable particles from the connecting member into the bloodstream.

From the above, it can be seen that a variety of approaches to placing embolic devices have been developed, but all of them are limited in some way by the time to release, the dispersion of particles or chemicals, the introduction of electricity, mechanical force on the implant after placement, or some combination of these affects. There is therefore a need for a precise, controlled method of deploying therapeutic interventional devices without compromising the position of the implant, without presenting broken or jagged ends that can potentially injure the vasculature, and without release undesirable particles or materials into the bloodstream.

Recently, a release system for vasoocclusive coils has been developed involving the use of a microgripper made of shape memory material. The shape memory micro grippers is mechanically actuated by the conversion of laser light to heat energy. Another newly developed type of release mechanism using shape memory materials involves a tube of radially recovering shape memory polymer attached to the distal end of an optical fiber pusher. A device such as an endoluminal coil is introduced into the tube and the tube is compressed or crimped around the end of the coil to hold it in place. Once the coil is in the desired location in the vasculature, the tube of shape memory polymer is heated by passing light through the optical fiber pusher to the distal end of the pusher, thereby causing the tube to recover its original diameter and shape. After the tube has recovered its original shape, it is no longer compressed or crimped around the device and the device is free to slip out of the tube.

US 5,609,608 discloses a miniature plastic gripper actuated by inflation of a miniature balloon. The gripper is constructed of either heat-shrinkable or heat-expandable plastic tubing. The gripper is connected at one end with a catheter or tube having an actuating balloon at its tip, the gripper being opened or closed by inflation or deflation of the balloon.

In another approach, an endoluminal coil delivery system is provided with a mechanical release mechanism for positioning and delivering a coil within a lumen that utilizes a mechanical latch to engage the coil during positioning. The coil is placed at the distal end of delivery system and includes a fitting at the end of coil which is engaged by jaws. The coil is released from the jaws by advancing a release tube over the jaws, which squeezes the jaws, thereby disengaging them from the fitting.

Another endoluminal coil delivery system utilizes an elongated pusher member with a coil implant detachably coupled to a relatively short flexible distal section of the pusher member by a curved coupling portion of the pusher member that can be thermally activated to transform to a preset release configuration. In another coil delivery system, embolic coils are threaded onto a guide wire ahead of a pusher located within a catheter, allowing several coils to be loaded on the guide wire. In another endoluminal coil delivery system, an end of a vasoocclusive coil is open and adapted to receive and engage a conical or cylindrical tip of a pusher core wire. The tip of the pusher wire may be smooth, grooved or in a semi-machined or sanded condition to achieve good adherence of the pusher core wire to the interior of the vasoocclusive coil.

A shape memory metal actuated separation device is also known that can be used for spacecraft. A segmented nut engages a threaded bolt that is to be held and released and is held together by a nut retainer that is movable with respect to the nut and affixed to a shape memory alloy element. The shape memory alloy element is heated by an electrical resistance heater, thereby moving the retainer which causes disengagement.

In one coil shaped intravascular stent formed into a coil spring, to be used to reinforce an arterial wall, the wire forming the stent has axially spaced rollers or bearings to facilitate advancement and withdrawal of the coil spring, with enlarged beads between the rollers to hold the rollers away from one another.

Thus, it can be seen that there is a continued requirement for reliable vascular device and embolic coil release systems. The present invention meets these and other needs.

### SUMMARY OF THE INVENTION

Briefly, and in general terms, the present invention provides for an improved system and method for release and deployment of an endoluminal therapeutic device at a desired location for treatment within the vasculature of a patient, utilizing a yieldable material for releasably holding a portion of the endoluminal therapeutic device, to be dislodged from the yieldable material at the desired location for treatment within the vasculature of a patient.

According to the present invention there is provided an endoluminal device delivery assembly for release and deployment of an endoluminal therapeutic device at a desired location for treatment within the vasculature of a patient, the endoluminal device having proximal and distal portions, the delivery device comprising an elongated flexible tubular catheter having a frustoconical distal end, and a tubular distal tip having a frustoconical shape corresponding to the frustoconical distal end of the elongated flexible tubular catheter, a proximal end mounted to the distal end of the catheter, an inner lumen, and a distal end with a surface defining a distal opening, and the tubular distal tip being formed of a yieldable material for releasably holding the proximal end of the endoluminal device within the inner lumen of the tubular distal tip and means for dislodging the proximal end of the endoluminal device from the inner lumen of the tubular distal tip to expel the proximal end of the endoluminal device through the distal opening of the tubular distal tip at the desired location for treatment within the vasculature of a patient.

In one presently preferred embodiment, the means for dislodging the proximal end of the endoluminal device from the inner lumen of the tubular distal tip comprises an elongated pusher member coaxially disposed within the elongated flexible tubular catheter having proximal and distal ends, with the proximal end of the pusher member extending from the proximal end of the elongated flexible tubular catheter, and the distal end of the pusher member being adapted to contact and dislodge the proximal end of the endoluminal device from the tubular distal tip.

In one preferred alternate embodiment, a flexible coil is mounted to the distal end of the elongated pusher member. The flexible coil can be formed from a shape memory polymer, a nickel titanium alloy, stainless steel, platinum, or other similar suitable materials.

In another presently preferred embodiment, the tubular distal tip forms a fluid seal about the proximal end of the endoluminal device, and the means for dislodging comprises a syringe connectable to the proximal end of the elongated flexible tubular catheter for supplying pressurized fluid within the elongated flexible tubular catheter to expel the proximal end of the endoluminal device from the tubular distal tip.

In one presently preferred embodiment, the diameter of the distal end of the tubular distal tip is smaller than the proximal end, allowing the proximal end of the endoluminal device to be captured within the inner lumen of the tubular distal tip. The yieldable material forming the tubular distal tip can comprise a shape memory material, such as a shape memory polymer, a nickel titanium alloy, an elastomer such as polyurethane, nylon, polybutyl terephthalate (PBT), polymers available under the trade names PEBAX, Hytrel, Arnitel, Riteflex, or other similar suitable yieldable materials.

The endoluminal therapeutic device advantageously has a stem portion with an elongated proximal end captured within the inner lumen of the tubular distal tip, and is typically an embolic coil, although the endoluminal therapeutic device can also be a stent, intravascular vena cava filter, or similar device to be implanted at a treatment site in the vasculature of a patient.

The endoluminal therapeutic device may be delivered into the vasculature of a patient by the steps of providing an elongated flexible tubular catheter having a tubular distal tip mounted to the distal end of the catheter, the tubular distal tip having an inner lumen and a distal end with a surface defining a distal opening, and the tubular distal tip being formed of a yieldable material for releasably holding the proximal end of the endoluminal device within the inner lumen of the tubular distal tip; and introducing a dislodging element into the proximal end of the elongated flexible catheter to dislodge the proximal end of the endoluminal device from the tubular distal tip to expel the proximal end of the endoluminal device through the distal opening of the tubular distal tip at the desired location for treatment within the vasculature of a patient. Preferably, the step of introducing a dislodging element comprises introducing an elongated pusher member coaxially within the elongated flexible tubular catheter to contact and dislodge the proximal end of the endoluminal device from the tubular distal tip. Alternatively, when the distal tip forms a fluid seal about the proximal end of the endoluminal device, and the step of introducing a dislodging element can comprise connecting a syringe to the proximal end of the elongated flexible tubular catheter for supplying pressurized fluid within the elongated flexible tubular catheter to expel the proximal end of the endoluminal device from the tubular distal tip.

These and other aspects and advantages of the invention will become apparent from the following detailed description and the accompanying drawings, which illustrate by way of example the features of the invention.

### BRIEF DESCRIPTION OF THE DRAWINGS

Figure 1 is a cross-sectional view of a first embodiment of the endoluminal device delivery assembly of the invention;
Fig. 2 is a side elevational view of a first embodiment of the pusher member of the endoluminal device delivery assembly of Fig. 1;
Fig. 3 is a side elevational view of an alternate embodiment of the pusher member of the endoluminal device delivery assembly of the invention; and
Fig. 4 is a sectional view of a syringe for use in combination with the catheter of the endoluminal device delivery assembly of the invention in another embodiment.

### DETAILED DESCRIPTION OF THE PREFERRED EMBODIMENTS

As very small medical instruments such as microcatheters have become available, physicians are now able to reach areas within the human body which were previously inaccessible. Among the areas which can now be accessed in a minimally invasive procedure are the tiny blood vessels within the brain. Using very small diameter pushers, it is now possible to insert therapeutic devices through microcatheters to treat damaged vasculature within the brain such as intracranial aneurysms. However, because the pushers and catheters used to deliver these devices are so small, there are practical limitations associated with their use. For example, because of the size and delicacy of the devices, it is not practical to have a device with complex moving parts at the distal end of the pusher, even though it is important to be able to reliably release the device from the pusher into the vasculature. While various methods for delivery of endoluminal devices to a treatment site within the vasculature have been developed, there remains a need for a reliable method of delivering and placing such devices, particularly into smaller, previously inaccessible areas of the vasculature.

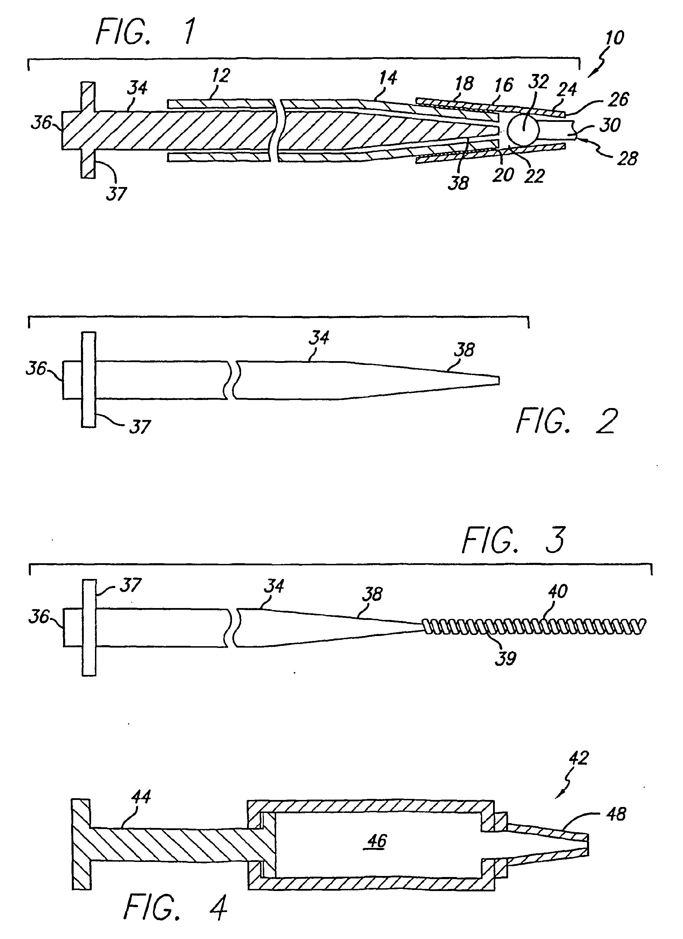

As is illustrated in the drawings, which are provided by way of example and not by way of limitation, the invention is embodied in an assembly for release and deployment of an endoluminal therapeutic device at a desired location for treatment within the vasculature of a patient. Referring to Fig. 1, the endoluminal device delivery assembly 10 includes an elongated flexible tubular catheter 12 having a distal end 14. The catheter can be formed, for example, from polyethylene, polyethylene terephthalate, polyvinyl chloride, nylon and ionomers, or other similar suitable polymers, stainless steel or nickel titanium alloy hypo tubes, and the like. The distal end of the elongated flexible tubular catheter has a frustoconical shape. The catheter advantageously includes a tubular distal tip 16 having a proximal end 18 mounted to the outer surface of the distal end of the catheter, such as by adhesive bonding, such as with a cyanoacrylate adhesive 20, for example. The tubular distal tip may alternatively be heat bonded to the distal end of the catheter, or may be mounted to the distal end of the catheter by other suitable means. The tubular distal tip has an inner lumen 22, and a distal end 24 with a surface defining a distal opening 26. In one presently preferred embodiment, the diameter of the distal end of the tubular distal tip is smaller than the proximal end, allowing the proximal end of the endoluminal device to be captured within the inner lumen of the tubular distal tip. The elongated flexible tubular catheter has a frustoconical distal end, and the distal tip has a corresponding frustoconical shape. In an alternative arrangement, a cylindrical tubular shape for the distal end of the catheter and the tubular distal tip may also be suitable.

The tubular distal tip is preferably formed of a yieldable material that is sufficiently rigid to retain the proximal end 28 of an endoluminal device within the inner lumen of the tubular distal tip. The yieldable material can be, for example, a shape memory polymer, an elastomer such as polyurethane, nylon, PEBAX polymer, Teloflex, polybutyl terephthalate (PBT), polymers available under the trade names PEBAX, Hytrel, Arnitel, Riteflex, heat shrink tubing such as polyethylene terephthalate (PET) or high density polyethylene (HDPE), or a shape memory metal such as nickel titanium alloy, such as that available under the trade name NITINOL

The proximal end of the endoluminal therapeutic device preferably has a stem portion 30 with an enlarged proximal end such as a ball 32, coil, block or the like, captured within the inner lumen of the tubular distal tip, and the endoluminal therapeutic device is typically an embolic coil, although the endoluminal therapeutic device can also be a stent, or a similar device to be implanted at a treatment site in the vasculature of a patient.

Means are also provided for dislodging the proximal end of the endoluminal device captured in the inner lumen of the tubular distal tip to expel the proximal end of the endoluminal device from the distal opening of the tubular distal tip at the desired location for treatment within the vasculature of a patient. As is illustrated in Figs. 1 and 2, in a presently preferred embodiment the means for dislodging the proximal end of the endoluminal device from the inner lumen of the tubular distal tip comprises an elongated flexible pusher member 34 such as a flexible metal wire coaxially disposed within the elongated flexible tubular catheter. The proximal end 36 of the pusher member extends from the proximal end of the elongated flexible tubular catheter, and preferably includes a stop portion 37 at the proximal end of the pusher member for limiting the movement of the pusher member through the delivery catheter, and the distal end 38 of the pusher member is adapted to contact and dislodge the proximal end of the endoluminal device from the tubular distal tip. As noted above, the distal end of the elongated flexible tubular catheter is narrowed, and has a frustoconical shape for improved tracking of the endoluminal device delivery assembly, and the distal end of the pusher member has a corresponding frustoconical shape, so as to extendable to the distal end of the catheter to force the proximal end of the endoluminal device from the yieldable tubular distal tip to dislodge the proximal end of the endoluminal device.

In an alternate preferred embodiment illustrated in Fig. 3, the distal end 38 of the pusher member 34 has a narrowed distal cylindrical tip 39, and a flexible coil 40 is mounted to the distal tip 39 of the elongated pusher member, such as by an adhesive such as cyanoacrylate, or heat bonding, and when the coil is formed of metal, by solder, welding, or the like. The flexible coil contacts the proximal end of the endoluminal device when the pusher member is moved distally through the delivery catheter, and reduces stiffness at the distal end of the pusher member, allowing for improved tracking of the endoluminal device delivery assembly within the vasculature, and can be formed from a shape memory polymer, a nickel titanium alloy, stainless steel, or platinum, for example, or other similar suitable materials.

In another presently preferred embodiment, the tubular distal tip is dimensioned so as to form a tight fluid seal about the proximal end of the endoluminal device, and the means for dislodging the endoluminal device comprises a syringe 42 having a plunger 44 for pressurizing fluid, such as saline, for example, in a fluid chamber 46 to supply pressurized fluid through a nozzle 48 that can be connected to the proximal end of the elongated flexible tubular catheter for supplying the pressurized fluid within the elongated flexible tubular catheter to expel the proximal end of the endoluminal device from the tubular distal tip.

In the method of delivering an endoluminal therapeutic device into the vasculature of a patient, the dislodging element, such as the pusher member, other similar mechanical device, or the pressurized fluid from the syringe, is introduced into the proximal end of the elongated flexible catheter to dislodge the proximal end of the endoluminal device from the tubular distal tip to expel the proximal end of the endoluminal device through the distal opening of the tubular distal tip at the desired location for treatment within the vasculature of a patient The elongated pusher member thus can be introduced and moved coaxially distally within the elongated flexible tubular catheter to contact and dislodge the proximal end of the endoluminal device from the tubular distal tip. Although a fluid seal need not be formed by the tubular distal tip over the proximal stem portion of the endoluminal device, when the distal tip is dimensioned to form a fluid seal about the proximal end of the endoluminal device, and a syringe or a similar device can be connected to the proximal end of the elongated flexible tubular catheter for supplying pressurized fluid within the elongated flexible tubular catheter to force the yieldable tubular distal tip of the catheter open to expel the proximal end of the endoluminal device from the tubular distal tip, to release the endoluminal device at the desired treatment site.

It will be apparent from the foregoing that while particular forms of the invention have been illustrated and described, various modifications can be made without departing from the scope of the invention. Accordingly, it is not intended that the invention be limited, except as by the appended claims.

## Claims

1. An endoluminal device delivery assembly (10) for release and deployment of an endoluminal therapeutic device at a desired location for treatment within the vasculature of a patient, the endoluminal therapeutic device having proximal and distal portions, the endoluminal device delivery assembly comprising:
an elongated flexible tubular catheter (12) having a frustoconical distal end (14);
a tubular distal tip (16) having a frustoconical shape corresponding to the frustoconical distal end (14) of the elongated flexible tubular catheter (12), a proximal end (18) mounted to the distal end of said catheter, an inner lumen (22), and a distal end (24) with a surface defining a distal opening (26), said tubular distal tip (16) being formed of a yieldable material for releasably holding the proximal end (28) of the endoluminal device within the inner lumen (22) of said tubular distal tip; and
means for dislodging the proximal end (28) of the endoluminal device from said inner lumen (22) of said tubular distal tip (16) to expel the proximal end (28) of the endoluminal device through said distal opening (26) of said tubular distal tip (16) at the desired location for treatment within the vasculature of a patient.

2. The endoluminal device delivery assembly (10) of Claim 1, wherein said means for dislodging comprises an elongated pusher member (34) coaxially disposed within the elongated flexible tubular catheter (12) having a proximal (36) and distal (38) ends, said proximal end (36) of said pusher member extending from said proximal end of said elongated flexible tubular catheter (12), and said distal end (38) of said pusher member being adapted to contact and dislodge said proximal end (28) of said endoluminal device from said tubular distal tip (16).

3. The endoluminal device delivery assembly (10) of Claim 2, wherein said distal end (38) of said pusher member (34) has a frustoconical shape.

4. The endoluminal device delivery assembly (10) of Claim 2, further comprising a flexible coil (40) mounted to the distal end (38) of said elongated pusher member (34).

5. The endoluminal device delivery assembly (10) of Claim 4, wherein said flexible coil (40) is formed from a shape memory polymer.

6. The endoluminal device delivery assembly (10) of Claim 4, wherein said flexible coil (40) is formed from a nickel titanium alloy.

7. The endoluminal device delivery assembly (10) of Claim 1, wherein said distal tip (16) forms a fluid seal about said proximal end (28) of said endoluminal device, and said means for dislodging comprises a syringe (42) connectable to said proximal end of said elongated flexible tubular catheter (12) for supplying pressurized fluid within said elongated flexible tubular catheter (12) to expel said proximal end (28) of said endoluminal device from said tubular distal tip (16).

8. The endoluminal device delivery assembly (10) of Claim 1, wherein the diameter of the distal end (24) of the distal tip (16) is smaller than the proximal end (18).

9. The endoluminal device delivery assembly (10) of Claim 1, wherein said yieldable material comprises a shape memory material.

10. The endoluminal device delivery assembly (10) of Claim 9, wherein said shape memory material is a shape memory polymer.

11. The endoluminal device delivery assembly (10) of Claim 9, wherein said shape memory material is a nickel titanium alloy.

12. The endoluminal device delivery assembly (10) of Claim 1, wherein said yieldable material is an elastomer.

13. The endoluminal device delivery assembly (10) of Claim 1, wherein said yieldable material is selected from the group consisting of polyurethane, nylon, and polybutyl terephthalate.

14. The endoluminal device delivery assembly (10) of Claim 1, wherein said endoluminal therapeutic device is an embolic coil.

15. The endoluminal device delivery assembly (10) of Claim 1, wherein said endoluminal therapeutic device has a stem portion (30) with an enlarged proximal end captured within said inner lumen (22) of said tubular distal tip (16).

## Patentansprüche

1. Einbringungsanordnung für eine Endoluminalvorrichtung (10) für die Freigabe und Entfaltung einer therapeutischen Endoluminalvorrichtung an einer gewünschten Stelle für die Behandlung in dem Gefäßsystem eines Patienten, wobei die therapeutische Endoluminalvorrichtung einen proximalen und einen distalen Teil aufweist, wobei die Einbringungsanordnung für eine Endoluminalvorrichtung umfasst:
einen länglichen, flexiblen, röhrenförmigen Katheter (12) mit einem kegelstumpfförmigen distalen Ende (14);
eine röhrenförmige distale Spitze (16) mit einer kegelstumpfförmigen Gestalt, die dem kegelstumpfförmigen distalen Ende (14) des länglichen, flexiblen, röhrenförmigen Katheters (12) entspricht, ein an dem distalen Ende des Katheters angebrachtes proximales Ende (18), ein inneres Lumen (22) und ein distales Ende (24) mit einer Fläche, die eine distale Öffnung (26) begrenzt, wobei die röhrenförmige distale Spitze (16) aus einem nachgiebigen Material gebildet ist, um das proximale Ende (28) der Endoluminalvorrichtung in dem inneren Lumen (22) der röhrenförmigen distalen Spitze lösbar zu halten; und
ein Mittel zum Entfernen des proximalen Endes (28) der Endoluminalvorrichtung aus dem inneren Lumen (22) der röhrenförmigen distalen Spitze (16) zum Ausstoßen des proximalen Endes (28) der Endoluminalvorrichtung durch die distale Öffnung (26) der röhrenförmigen distalen Spitze (16) an der gewünschten Stelle für die Behandlung in dem Gefäßsystem eines Patienten.

2. Einbringungsanordnung für eine Endoluminalvorrichtung (10) nach Anspruch 1, wobei das Mittel zum Entfernen ein längliches Schieberelement (34) umfasst, das koaxial in dem länglichen, flexiblen, röhrenförmigen Katheter (12) angebracht ist und ein proximales (36) und ein distales (38) Ende aufweist, wobei sich das proximale Ende (36) des Schieberelements von dem proximalen Ende des länglichen, flexiblen, röhrenförmigen Katheters (12) erstreckt und das distale Ende (38) des Schieberelements dafür ausgelegt ist, das proximale Ende (28) der Endoluminalvorrichtung von der röhrenförmigen, distalen Spitze (16) zu entfernen.

3. Einbringungsanordnung für eine Endoluminalvorrichtung (10) nach Anspruch 2, wobei das distale Ende (38) des Schieberelements (34) eine kegelstumpfförmige Gestalt aufweist.

4. Einbringungsanordnung für eine Endoluminalvorrichtung (10) nach Anspruch 2, welche ferner eine flexible Spule (40) aufweist, die an dem distalen Ende (38) des länglichen Schieberelements (34) angebracht ist.

5. Einbringungsanordnung für eine Endoluminalvorrichtung (10) nach Anspruch 4, wobei die flexible Spule (40) aus einem Formgedächtnispolymer gebildet ist.

6. Einbringungsanordnung für eine Endoluminalvorrichtung (10) nach Anspruch 4, wobei die flexible Spule (40) aus einer Nickel-Titan-Legierung gebildet ist.

7. Einbringungsanordnung für eine Endoluminalvorrichtung (10) nach Anspruch 1, wobei die distale Spitze (16) eine Fluiddichtung an dem proximalen Ende (28) der Endoluminalvorrichtung bildet, und wobei das Mittel zum Entfernen eine Spritze (42) umfasst, die mit dem proximalen Ende des länglichen, flexiblen, röhrenförmigen Katheters (12) verbunden werden kann, um unter Druck stehendes Fluid in dem länglichen, flexiblen, röhrenförmigen Katheter (12) bereitzustellen, um das proximale Ende (28) der Endoluminalvorrichtung von der röhrenförmigen, distalen Spitze (16) auszustoßen.

8. Einbringungsanordnung für eine Endoluminalvorrichtung (10) nach Anspruch 1, wobei der Durchmesser des distalen Endes (24) der distalen Spitze (16) kleiner ist als das proximale Ende (18).

9. Einbringungsanordnung für eine Endoluminalvorrichtung (10) nach Anspruch 1, wobei das nachgiebige Material ein Formgedächtnismaterial umfasst.

10. Einbringungsanordnung für eine Endoluminalvorrichtung (10) nach Anspruch 9, wobei das Formgedächtnismaterial ein Formgedächtnispolymer ist.

11. Einbringungsanordnung für eine Endoluminalvorrichtung (10) nach Anspruch 9, wobei das Formgedächtnismaterial eine Nickel-Titan-Legierung ist.

12. Einbringungsanordnung für eine Endoluminalvorrichtung (10) nach Anspruch 1, wobei das nachgiebige Material ein Elastomer ist.

13. Einbringungsanordnung für eine Endoluminalvorrichtung (10) nach Anspruch 1, wobei das nachgiebige Material gewählt ist aus der Gruppe, umfassend Polyurethan, Nylon und Polybutylterephthalat.

14. Einbringungsanordnung für eine Endoluminalvorrichtung (10) nach Anspruch 1, wobei die therapeutische Endoluminalvorrichtung eine embolische Spule ist.

15. Einbringungsanordnung für eine Endoluminalvorrichtung (10) nach Anspruch 1, wobei die therapeutische Endoluminalvorrichtung einen Stammabschnitt (30) mit einem vergrößerten proximalen Ende umfasst, das in dem inneren Lumen (22) der röhrenförmigen distalen Spitze (16) gefangen ist.

## Revendications

1. Ensemble de délivrance de dispositif endoluminal (10) destiné à la libération et au déploiement d'un dispositif endoluminal thérapeutique à un site souhaité pour le traitement à l'intérieur du système vasculaire d'un patient, le dispositif endoluminal thérapeutique ayant des parties proximale et distale, l'ensemble de délivrance de dispositif endoluminal comprenant :
un cathéter tubulaire souple allongé (12) ayant une extrémité distale tronconique (14),
une pointe distale tubulaire (16) ayant une forme tronconique correspondant à l'extrémité distale tronconique (14) du cathéter tubulaire souple allongé (12), une extrémité proximale (18) montée sur l'extrémité distale dudit cathéter, une lumière interne (22) et une extrémité distale (24) ayant une surface définissant une ouverture distale (26), ladite pointe distale tubulaire (16) étant formée d'un matériau malléable destiné à maintenir avec possibilité de libération l'extrémité proximale (28) du dispositif endoluminal à l'intérieur de la lumière interne (22) de ladite pointe distale tubulaire, et
un moyen destiné à déloger l'extrémité proximale (28) du dispositif endoluminal depuis ladite lumière interne (22) de ladite pointe distale tubulaire (16) afin d'expulser l'extrémité proximale (28) du dispositif endoluminal au travers de ladite ouverture distale (26) de ladite pointe distale tubulaire (16) au site souhaité pour un traitement à l'intérieur du système vasculaire d'un patient.

2. Ensemble de délivrance de dispositif endoluminal (10) selon la revendication 1, dans lequel ledit moyen destiné au détachage comprend un élément de poussée allongé (34) disposé de façon coaxiale à l'intérieur du cathéter tubulaire souple allongé (12) ayant une extrémité proximale (36) et une extrémité distale (38), ladite extrémité proximale (36) dudit élément de poussée s'étendant depuis ladite extrémité proximale dudit cathéter tubulaire souple allongé (12), et ladite extrémité distale (38) dudit élément de poussée étant conçue pour venir en contact avec ladite extrémité proximale (28) dudit dispositif endoluminal, et déloger celle-ci de ladite pointe distale tubulaire (16).

3. Ensemble de délivrance de dispositif endoluminal (10) selon la revendication 2, dans lequel ladite extrémité distale (38) dudit élément de poussée (34) a une forme tronconique.

4. Ensemble de délivrance de dispositif endoluminal (10) selon la revendication 2, comprenant en outre un enroulement souple (40) monté sur l'extrémité distale (38) dudit élément de poussée allongé (34).

5. Ensemble de délivrance de dispositif endoluminal (10) selon la revendication 4, dans lequel ledit enroulement souple (40) est formé à partir d'un polymère à mémoire de forme.

6. Ensemble de délivrance de dispositif endoluminal (10) selon la revendication 4, dans lequel ledit enroulement souple (40) est formé à partir d'un alliage de nickel et de titane.

7. Ensemble de délivrance de dispositif endoluminal (10) selon la revendication 1, dans lequel ladite pointe distale (16) forme un joint étanche aux fluides autour de ladite extrémité proximale (28) dudit dispositif endoluminal et ledit moyen destiné au détachage comprend une seringue (42) pouvant être reliée à ladite extrémité proximale dudit cathéter tubulaire souple allongé (12) afin de fournir le fluide sous pression à l'intérieur dudit cathéter tubulaire souple allongé (12) pour expulser ladite extrémité proximale (28) dudit dispositif endoluminal depuis ladite pointe distale tubulaire (16).

8. Ensemble de délivrance de dispositif endoluminal (10) selon la revendication 1, dans lequel le diamètre de l'extrémité distale (24) de la pointe distale (16) est plus petit que celui de l'extrémité proximale (18).

9. Ensemble de délivrance de dispositif endoluminal (10) selon la revendication 1, dans lequel ledit matériau malléable comprend un matériau à mémoire de forme.

10. Ensemble de délivrance de dispositif endoluminal (10) selon la revendication 9, dans lequel ledit matériau à mémoire de forme est un polymère à mémoire de forme.

11. Ensemble de délivrance de dispositif endoluminal (10) selon la revendication 9, dans lequel ledit matériau à mémoire de forme est un alliage de nickel et de titane.

12. Ensemble de délivrance de dispositif endoluminal (10) selon la revendication 1, dans lequel ledit matériau malléable est un élastomère.

13. Ensemble de délivrance de dispositif endoluminal (10) selon la revendication 1, dans lequel ledit matériau malléable est sélectionné à partir du groupe constitué de polyuréthanne, de Nylon et de polytéréphtalate de butyle.

14. Ensemble de délivrance de dispositif endoluminal (10) selon la revendication 1, dans lequel ledit dispositif endoluminal thérapeutique est un serpentin pour embolie.

15. Ensemble de délivrance de dispositif endoluminal (10) selon la revendication 1, dans lequel ledit dispositif endoluminal thérapeutique a une partie de tige (30) ayant une extrémité proximale élargie capturée à l'intérieur de ladite lumière interne (22) de ladite pointe distale tubulaire (16).
